# EUROPÄISCHE PATENTSCHRIFT

(11) **EP 0 854 859 B1**
(45) Veröffentlichungstag und Bekanntmachung des Hinweises auf die Patenterteilung: **24.05.2000**
(21) Anmeldenummer: 96928476.9
(22) Anmeldetag: 19.08.1996
(51) Int. Cl.: C07C 253/30

(54) **VERFAHREN ZUR HERSTELLUNG VON 4-CYANO-2,5-DIFLUORANILIN**
4-CYANO-2,5-DIFLUORANILINE PREPARATION PROCESS
PROCEDE DE PREPARATION DE 4-CYANO-2,5-DIFLUORANILINE

(30) Priorität: 30.08.1995 DE 19531895
(43) Veröffentlichungstag der Anmeldung: 29.07.1998
(73) Patentinhaber: BAYER AG, 51368 Leverkusen (DE)
(72) Erfinder: MARHOLD, Albrecht, D-51373 Leverkusen (DE); BIELEFELDT, Dietmar, D-40883 Ratingen (DE); GALLENKAMP, Bernd, D-42113 Wuppertal (DE)
(86) Internationale Anmeldenummer: EP9603642
(87) Internationale Veröffentlichungsnummer: WO9708136

(56) Entgegenhaltungen:
- EP-A- 0 224 001
- EP-A- 0 415 595
- EP-A- 0 497 213
- JOURNAL OF MEDICINAL CHEMISTRY, Bd. 37, Nr. 4, 18.Februar 1994, WASHINGTON US, Seiten 467-475, XP002020660 J. OHMORI ET AL: "6-(1-H-Imidazol-1-yl)-7-nitro-2,3(1H,4H)- quinoxalinedione hydrochloride (YM90K) and related compounds: Structure-activity relationships for the AMPA-type non-NMDA receptor"

## Beschreibung

Die vorliegende Erfindung betrifft ein Verfahren zur Herstellung von 4-Cyano-2,5-difluoranilin, welches ein Zwischenprodukt für die Herstellung von Herbiziden oder Flüssigkristallen ist.

Das bekannte zweistufige Verfahren zur Herstellung von 4-Cyano-2,5-difluoranilin geht von 2,5-Difluoranilin aus (siehe EP-A1 0 224 001). In der ersten Stufe wird zum 4-Brom-2,5-difluoranilin bromiert. In der zweiten Stufe wird das Brom gegen Cyano ausgetauscht. Hierzu wird, neben einer gut äquimolaren Menge Kupfercyanid für die Reaktion, eine ca. 5-fach molare Menge 10 %ige Natriumcyanid-Lösung zur Aufarbeitung benötigt. Von erheblichem Nachteil sind die Kosten für Kupfercyanid und die, nicht nur wegen des als Cyano-Komplex gebundenen Kupfers, problematische Abwasseraufarbeitung, die langwierige extraktive Aufarbeitung, insbesondere die große benötigte Menge Dichlormethan, sowie die Handhabung großer Mengen sehr giftiger Natriumcyanid-Lösung.

Es wurde gefunden, daß man 4-Cyano-2,5-difluoranilin in hoher Ausbeute und Reinheit erhält, wenn man 2,4,5-Trifluorbenzonitril mit einem Überschuß von Ammoniak gegebenenfalls in Gegenwart eines Verdünnungsmittels bei erhöhten Temperaturen umsetzt.

Überraschenderweise führt auch ein großer Überschuß von Ammoniak nicht zu einem Di- oder Triamin. Dies gilt besonders im Hinblick auf bereits beschriebene Verfahren zur Herstellung von 3,5-Difluoranilin (EP-A 497 213) oder o-Nitroanilin (Ohmori et al., Journal of Medicinal Chemistry (1994), 37, 467-475). Darüberhinaus ist es sehr überraschend, daß selektiv der Austausch nur in 4-Position und nicht in 2-Position erfolgt. Führt man analoge Umsetzungen mit 2,4-Difluorbenzonitril unter den erfindungsgemäßen Bedingungen durch, werden die beiden möglichen Amine etwa im Verhältnis 1:1 gebildet.

Das als Ausgangsstoff benötigte 2,4,5-Trifluorbenzonitril ist kommerziell verfügbar und kann z.B. aus 2,4-Dichlor-5-fluorbenzoylchlorid durch Fluorierung, Amidierung und folgende Dehydratisierung hergestellt werden.

Als Verdünnungsmittel zur Durchführung der erfindungsgemäßen Umsetzung kommen Wasser, bestimmte organische Lösungsmittel und beliebige Mischungen davon in Betracht. Beispielhaft seien genannt: aliphatische, alicyclische oder aromatische Kohlenwasserstoffe, wie beispielsweise Petrolether, Hexan, Heptan, Cyclohexan, Methylcyclohexan, Benzol, Toluol, Xylol oder Decalin; halogenierte Kohlenwasserstoffe, wie beispielsweise Chlorbenzol, Dichlorbenzol; Ether, wie Diethylether, Diisopropylether-, Methyl-t-butylether-, Methyl-t-amylether, Dioxan, Tetrahydrofuran, 2-Methyltetrahydrofuran, 1,2-Dimethoxyethan, Diethylenglycoldimethylether, 1,2-Diethoxyethan oder Anisol. Bevorzugt ist prinzipiell die Verwendung eines Verdünnungsmittel. Bevorzugt als Lösungsmittel sind Dioxan, Tetrahydrofuran, Toluol oder Wasser.

Die Umsetzung wird bei Temperaturen von 50 bis 150°C, vorzugsweise von 80 bis 130°C durchgeführt.

Die Umsetzung wird im allgemeinen bei erhöhtem Druck durchgeführt. Bevorzugt ist die Durchführung bei Drücken, die mindestens dem Eigendruck der Reaktionsmischung bei der Reaktionstemperatur entsprechen. Besonders bevorzugt wird ein Überdruck von 2 bis 50 bar.

Bei der Durchführung der erfindungsgemäßen Umsetzung setzt man pro Mol 2,4,5-Trifluorbenzonitril im allgemeinen 1,5 bis 50 Mol, vorzugsweise 2 bis 20 Mol Ammoniak und gegebenenfalls 0,1 bis 3 l, vorzugsweise 0,3 bis 2 l Verdünnungsmittel ein.

In einer bevorzugten Ausführungsform des Umsetzungsschrittes des erfindungsgemäßen Verfahrens wird das Nitril mit dem Verdünnungsmittel in einem verschlossenen Reaktionsgefäß vorgelegt und auf Reaktionstemperatur aufgeheizt. Dann wird flüssiger Ammoniak gegen den Behälterdruck so hinzugepumpt, daß die Innentemperatur konstant gehalten werden kann.

In einer bevorzugten Ausführungsform des Aufarbeitungsschrittes des erfindungsgemäßen Verfahrens wird nach Beendigung der Umsetzung abgekühlt, der Behälter entspannt und der Überschuß Ammoniak und das Verdünnungsmittel gegebenenfalls unter vermindertem Druck entfernt. Zur Isolierung des 4-Cyano-2,5-difluoranilins wird der Rückstand mit Wasser aufgerührt, die Kristalle abgetrennt, mit Wasser aufgeschlämmt und erneut abgetrennt und zuletzt getrocknet.

Das nach dem erfindungsgemäßen Verfahren herstellbare 4-Cyano-2,5-difluoranilin kann z.B. als Zwischenprodukt für die Herstellung von Herbiziden oder Flüssigkristallen verwendet werden (siehe z.B. EP-A1 648 772; DE-OS 38 351 68; JP 02 243 676).

### Beispiel 1

In einem Autoklaven werden 515 g 2,4,5-Trifluorbenzonitril in 2000 ml Tetrahydrofuran vorgelegt und auf 100°C erhitzt. Dann werden 250 ml flüssiger Ammoniak zugepumpt, wobei die Dosierung so erfolgt, daß die Temperatur im Bereich von 100 bis 105°C bleibt. Bei dieser Temperatur wird für 8 h nachgerührt und dann abgekühlt. Der Überdruck erreicht maximal 22 bar. Nach Abblasen des Ammoniaks wird das Lösungsmittel mit einem Rotationsverdampfer entfernt. Der Rückstand wird mit 500 ml Wasser aufgerührt und auf eine Nutsche verbracht. Nach Absaugen wird nochmals mit 200 ml Wasser angeschlämmt und abgesaugt. Nach Trocknung im Vakuum bei 40°C verbleiben 482 g (95,4 % d.Theorie) an 4-Cyano-2,5-difluor-anilin mit einem Schmelzpunkt von 99 bis 100°C.

### Beispiel 2

In einem mit einem Ankerrührer ausgerüsteten 40 l VA-Autoklaven addiert man bei ca. 20°C zu einer Lösung von 3000 g 2,3,5-Trilfuorbenzonitril in 10 l Methyl-tert.-butylether 4500 ml flüssigen Ammoniak. Zu dem Eigendruck des Reaktionsgemisches drückt man 10 bar Stickstoff auf und setzt das Gemisch 16 Stunden lang bei 100°C um. Nach Entfernen des nicht umgesetzten Ammoniaks und der Extraktion des Lösungsmitels mit Wasser destillieret man das Lösungsmittel bei 40 bis 60°C (200-300 mbar) ab. man erhält als Rückstand 2839 g reines Produkt. Dies entspricht einer Ausbeute von 96,5 % d.Th..

## Patentansprüche

1. Verfahren zur Herstellung von 4-Cyano-2,5-difluoranilin, dadurch gekennzeichnet, daß man 2,4,5-Trifluorbenzonitril mit einem Überschuß von Ammoniak gegebenenfalls in Gegenwart eines Verdünnungsmittels bei erhöhter Temperatur umsetzt.

2. Verfahren zur Herstellung von 4-Cyano-2,5-difluoranilin gemäß Anspruch 1, dadurch gekennzeichnet, daß man die Umsetzung bei Temperaturen von 50 bis 150°C durchführt.

3. Verfahren zur Herstellung von 4-Cyano-2,5-difluoranilin gemäß Anspruch 1, dadurch gekennzeichnet, daß man die Umsetzung bei erhöhtem Druck durchführt.

4. Verfahren zur Herstellung von 4-Cyano-2,5-difluoranilin gemäß Anspruch 1, dadurch gekennzeichnet, daß man bei der Umsetzung pro Mol 2,4,5-Trifluorbenzonitril 1,5 bis 50 Mol Ammoniak und gegebenenfalls 0,1 bis 3 l Verdünnungsmittel einsetzt.

5. Verfahren zur Herstellung von 4-Cyano-2,5-difluoranilin gemäß Anspruch 1, dadurch gekennzeichnet, daß man das Nitril mit dem Verdünnungsmittel in einem verschlossenen Reaktionsgefäß vorlegt, auf die Reaktionstemperatur aufheizt, den flüssigen Ammoniak gegen den Behälterdruck so hinzupumpt, daß die Innentemperatur gehalten wird, nach Beendigung der Umsetzung abkühlt, den Überschuß Ammoniak und das Verdünnungsmittel gegebenenfalls unter vermindertem Druck entfernt, den Rückstand mit Wasser aufrührt, die Kristalle abtrennt, mit Wasser wäscht oder in Wasser aufschlämmt, erneut abtrennt und zuletzt trocknet.

## Claims

1. Process for preparing 4-cyano-2,5-difluoroaniline, characterized in that 2,4,5-trifluorobenzonitrile is reacted with an excess of ammonia at elevated temperature, optionally in the presence of a diluent.

2. Process for preparing 4-cyano-2,5-difluoroaniline according to Claim 1, characterized in that the reaction is carried out at temperatures of from 50 to 150°C.

3. Process for preparing 4-cyano-2,5-difluoroaniline according to Claim 1, characterized in that the reaction is carried out at increased pressure.

4. Process for preparing 4-cyano-2,5-difluoroaniline according to Claim 1, characterized in that the reaction uses from 1.5 to 50 mol of ammonia and, if desired, from 0.1 to 3 1 of diluent per mole of 2,4,5-trifluorobenzonitrile.

5. Process for preparing 4-cyano-2,5-difluoroaniline according to Claim 1, characterized in that the nitrite, with the diluent, is introduced into a sealed reaction vessel and heated to the reaction temperature, the liquid ammonia is pumped in against the container pressure at such a rate that the internal temperature is maintained, then after the reaction is complete the system is cooled, the excess ammonia and the diluent are removed, if necessary under reduced pressure, the residue is stirred with water, and the crystals are separated off, washed with water or slurried in water, separated off again and finally dried.

## Revendications

1. Procédé de préparation de 4-cyano-2,5-difluoroaniline, caractérisé en ce que l'on fait réagir le 2,4,5-trifluorobenzonitrile avec un excès d'ammoniac, facultativement en présence d'un agent de dilution, à températures élevées.

2. Procédé de préparation de 4-cyano-2,5-difluoroaniline suivant la revendication 1, caractérisé en ce que l'on réalise la réaction à des températures allant de 50 à 150°C.

3. Procédé de préparation de 4-cyano-2,5-difluoroaniline suivant la revendication 1, caractérisé en ce que l'on réalise la réaction à pression élevée.

4. Procédé de préparation de 4-cyano-2,5-difluoroaniline suivant la revendication 1, caractérisé en ce que l'on met en oeuvre pour la réaction, par mole de 2,4,5-trifluorobenzonitrile, 1,5 à 50 moles d'ammoniac et facultativement, 0,1 à 3 litres d'agent de dilution.

5. Procédé de préparation de 4-cyano-2,5-difluoroaniline suivant la revendication 1, caractérisé en ce que l'on dispose le nitrile avec l'agent de dilution dans un récipient de réaction fermé, on chauffe à la température de réaction, on pompe alors l'ammoniac liquide, contre la pression du récipient, de sorte que la température interne soit maintenue, après achèvement de la réaction, on refroidit, on élimine l'excès d'ammoniac et l'agent de dilution, facultativement sous pression réduite, on reprend le résidu dans de l'eau, on sépare les cristaux, on lave à l'eau ou on remet en suspension dans de l'eau, on sépare à nouveau et finalement, on sèche.
